# EUROPÄISCHE PATENTSCHRIFT

(11) **EP 1 337 624 B1**
(45) Veröffentlichungstag und Bekanntmachung des Hinweises auf die Patenterteilung: **20.09.2006**
(21) Anmeldenummer: 01996595.3
(22) Anmeldetag: 07.11.2001
(51) Int. Cl.: C12N 5/06, C12N 5/00

(54) **ZELLKONSTRUKTE ERHÄLTLICH AUS MESENCHYMALEN STAMMZELLEN UND DAVON ABLEITBAREN ZELLEN UND IHRE VERWENDUNG**
CELL CONSTRUCTS WHICH CAN BE OBTAINED FROM MESENSCHYMAL STEM CELLS AND CELLS DERIVABLE THEREFROM AND THE USE THEREOF
CONSTRUCTIONS CELLULAIRES OBTENUES A PARTIR DE CELLULES SOUCHES MESENCHYMATEUSES, CELLULES POUVANT DERIVER DE CELLES-CI ET LEUR UTILISATION

(30) Priorität: 14.11.2000 DE 10056465
(43) Veröffentlichungstag der Anmeldung: 27.08.2003
(73) Patentinhaber: Daig, Rosemarie, 93053 Regensburg (DE)
(72) Erfinder: Daig, Rosemarie, 93053 Regensburg (DE)
(74) Vertreter: Ackermann, Joachim
(86) Internationale Anmeldenummer: PCT/EP2001/012852
(87) Internationale Veröffentlichungsnummer: WO 2002/040645

(56) Entgegenhaltungen:
- WO-A-95/01423
- US-A- 5 486 359
- US-A- 5 591 625
- US-A- 5 800 811
- US-A- 5 968 546
- REYNOLDS A J ET AL: "HAIR FOLLICLE STEM CELLS? A DISTINCT GERMINATIVE EPIDERMAL CELL POPULATION IS ACTIVATED IN VITRO BY THE PRESENCE OF HAIR DERMAL PAPILLA CELLS" JOURNAL OF CELL SCIENCE, ESSEX, GB, Bd. 99, Nr. 2, Juni 1991 (1991-06), Seiten 373-385, XP001010318
- YOUNG RANDELL G ET AL: "Use of mesenchymal stem cells in a collagen matrix for achilles tendon repair." JOURNAL OF ORTHOPAEDIC RESEARCH, Bd. 16, Nr. 4, Juli 1998 (1998-07), Seiten 406-413, XP002204291 ISSN: 0736-0266

## Beschreibung

Die Erfindung betrifft Zellkonstrukte erhältlich aus mesenchymalen Stammzellen und von diesen ableitbaren Zellen des Bindegewebes, wie z. B. Knochen-, Knorpel-, Fett- oder Muskelzellen, die Verwendung solcher Zellkonstrukte sowie Verfahren zu ihrer Herstellung und Verwendung.

Die Erzeugung von differenzierten Geweben aus pluripotenten Stammzellen, z. B. zur Herstellung von Transplantaten, ist seit längerem Gegenstand intensiver Entwicklungsarbeit. Grundlage dieser. Bemühungen ist die Fähigkeit solcher Stammzellen sich durch wiederholte mitotische Teilung selbst zu erneuern sowie die Fähigkeit dieser Stammzellen, in Abhängigkeit bestimmter Faktoren, sich in morphologisch und physiologisch unterschiedliche Zelltypen zu differenzieren.

So ist die Differenzierung von Knochen-, Knorpel-, Muskel-, Fettgewebe, Dermis, Sehnen, Bändern und Knochenmark-Stromazellen aus mesenchymalen Stammzellen aus Periosteum und vor allem aus dem Knochenmark bereits gelungen (US 5,486,359, US 5,736,396).

In US 5,486,359, US 5,197,985, US 5,226,914, US 5,811,094, US 5,736,396, WO99/03973 und WO98/32450 sind z. B. vorteilhafte Methoden zur Implantation und Differenzierung von mesenchymalen Stammzellen aus dem Periosteum und aus dem Knochenmark zur Behandlung von Muskel-, Skelett-, Knorpel- oder anderen Bindegewebsschäden beschrieben. Dazu wird dem Patienten Knochenmark entnommen und in vitro auf mesenchymale Stammzellen über die Adhärenz und geeignetes Medium selektioniert. Nachteilig dabei ist, daß der Anteil an pluripotenten mesenchymalen Stammzellen im Knochenmark mit 0.001% bis 0.01% sehr gering ist. Deshalb müssen relative große Mengen an Knochenmark entnommen werden, um genügend Ausgangsmaterial für eine in vitro Kultur zu erhalten. Auch die Gewinnnung reiner Kulturen aus mesenchymalen Stammzellen und damit auch der davon abgeleiteten Bindegewebezellen ist aus diesem Grunde schwierig. Aufgrund des hohen Anteils anderer Zelltypen in der entnommenen Probe ist die Abtrennung der Stammzellen von Zellen anderen Typs über die spezifische Adhäranz von mesenchymalen Stammzellen bzw. Wahl des geeigneten Mediums problematisch. Als letztes ist die Probenentnahme durch Knochenmarkpunktation ein aufwendiger, für den Patienten mit einem gewissen Risiko behafteter Eingriff.

Darüber hinaus ist die für den Patienten risikolose Nutzung und Kultivierung epidermaler Stammzellen aus Haaren und deren Verwendung zur Behandlung von Hautdefekten bekannt (US 5,968,546). Demgegenüber wurde bisher, trotz großem Bedarf, noch kein für den Patienten risikoloses Verfahren zur Herstellung von mesenchymalen Stammzellen oder daraus ableitbarer Bindegewebstypen beschrieben.

Davon ausgehend liegt der Erfindung die Aufgabe zu Grunde einfach herstellbare Zellkonstrukte aus patientenschonend gewinnbarem mesenchymalen Zellmaterial oder daraus ableitbarer Gewebetypen sowie effiziente Verfahren zu ihrer Herstellung bereitzustellen.

Es kann überraschend gezeigt werden, dass Zellkonstrukte aus mesenchymalen Stammzellen und davon ableitbaren Geweben, einfach, mit aus Haarfollikeln isolierten mesenchymalen Stammzellen, die auf einer polymeren Matrix in vitro kultiviert werden, hergestellt werden können.

Somit betrifft die Erfindung Zellkonstrukte enthaltend eine Polymermatrix und mesenchymale Stammzellen aus Haarfollikeln oder davon durch Differenzierung ableitbarer Zellen.

Mesenchymale Stammzellen des Haarfollikels können z. B. durch Mikrodissektion oder überraschend auch durch Zupfen von Körperhaaren gewonnen werden.

Insbesondere ist das Zupfen eine schnelle, nicht-invasive Methode zur Gewinnung von mesenchymale Stammzellen des Haarfollikels . So können die Zellen einfach, ohne qualifiziertes medizinisches Personal, ohne Risiko und bei geringen Schmerzen für den Patienten entnommen werden.

Darüber hinaus ist der Anteil von mesenchymalen Stammzellen in der Haarwurzel sehr groß. Die aus den Haarwurzeln einfach isolierbaren Haarfollikel enthalten neben den mesenchymalen Stammzellen nur epitheliale Stammzellen in nennenswertem Umfang. Das ist die Grundlage für die Herstellung sehr reiner Gewebekulturen unter Verwendung einfacher Selektionsmethoden aus kleinen Probenmengen.

Um die Gewinnug mesenchymaler Stammzellen aus Haarfollikel durch Zupfen von Haaren, z. B. aus der Kopfhaut eines Probanden, zu ermöglichen, wird die Entnahmestelle mit einer die Haut aufweichenden Lösung behandelt. Danach können die Haare mit weitestgehend intaktem Haarfollikel mit einer Pinzette gezupft werden.

Um die mesenchymalen Stammzellen in Kultur zu nehmen werden die Haarfollikel vom Resthaar entfernt und in einer bevorzugten Ausführungsform kurz in einer antibakteriell wirkenden Lösung gebadet. Die Haarfollikel können auf einer festen oder gelartigen Adhäsionsoberfläche in Gegenwart eines FGF enthaltenden Mediums kultiviert werden, wobei bFGF der bevorzugt zu verwendende Faktor ist. Die Kultivierung erfolgt bei einer Temperatur zwischen 25 und 40°C, bis die Follikel auswachsen und konfluent werden. Als Medium kann z. B. Changs-Medium DMEM/Ham s F12, M199, E199, MEM, EMEM oder DMEM Grundmedium gegebenenfalls mit weiteren Zusätzen verwendet werden. Im Komplettmedium sollten bevorzugt folgende Mediumszusätze enthalten sein: bFGF, Serum, IGF-I, Insulin, Hydrocortison, Pyruvat, Retinsäure, nicht essentielle Aminosäuren, Triiodthyronin (T3), Transferrin, Selen und/oder Glucagon. Die Kultivierung der Haarfollikel erfolgt bevorzugt in feuchter Luft bei 37°C und 5% CO₂. Zur weiteren Kultivierung können die ausgewachsenen Zellen von der Oberfläche abgelöst und in einem geeigneten Medium, bevorzugt in obigem Medium propagiert werden.

Durch weitere Zusatzstoffe im Medium, wie z. B. das Wachstum von epithelialen Stammzellen spezifisch hemmende Faktoren, kann eine optimale Reinigung der mesenchymalen Stammzellen gewährleistet werden.

Die Zellkultur kann z. B. über immunolgische Separationsverfahren (FACS) oder immunologisch/magnetische Separation über an Beads befestigte Antikörper gereinigt werden. Dabei haben sich Antikörper bewährt, die sich gegen Antigene richten, die für mesenchymale Stammzellen spezifisch sind, wie z. B. die Antigene CD13, CD49e, CD29, CD105 oder das smooth-muscle-alpha-actin.

Ebenso besteht zur Reinigung der Zellkultur von epithelialen Stammzellen die Möglichkeit einer immunologischen Negativ-Selektion über CD24- oder Cytokeratin 16 Antikörper. Die Negativ-Selektion besitzt den Vorteil, daß Veränderungen in den mesenchymalen Stammzellen durch Antikörper induzierbare Signale wirksam vermieden werden und somit ein möglichst naiver Zustand der Zellen erhalten bleibt.

Alternativ oder additiv zu den eben beschriebenen Verfahren besteht allerdings auch die Möglichkeit die entnommenen und im Medium propagierten mesenchymalen Stammzellen aus Haarfollikeln über Dichtegradientenzentrifugation, z. B. in einem Percoll-Gradienten, von anderen Bestandteilen mit anderer Dichte insbesondere anderen Zellen, abzutrennen.

Über die immunologischen Seperationsverfahren lassen sich, ausgehend von einer im wesentlichen nur mesenchymale und epitheliale Stammzellen enthaltende Zellkultur, die schon während der Kultivierung vorselektioniert sein kann, hoch homogene Zellkulturen aus mesenchymalen Stammzellen gewinnen. Insbesondere die Herstellung von hoch homogenen Zellkulturen mit Negativ-Selektion ist aufgrund der definierten Zusammensetzung des für die Selektion verwendeten Ausgangszellmaterials möglich.

Die so gewonnenen und aufgereinigten mesenchymalen Stammzellen können nun zur Herstellung von Zellkonstrukten verwendet werden.
Dazu werden die mesenchymale Stammzellen in einem geeigneten Medium suspendiert und bei einer Temperatur zwischen 25 und 40 °C auf eine Polymermatrix aufgebracht. Bevorzugt kann direkt die aus der Reinigung der Zellkultur erhaltene Suspension verwendet werden.

Als Matrix kommen auf die mesenchymalen Stammzellen adhäsiv bzw. immobilisierend wirkende feste oder gelförmige Polymere in Frage, wobei die Matrixbildung gelierender Polymere bevorzugt zu Beginn der Zellkonstruktherstellung nach Inkubation mit den Stammzellen erfolgt. Synthetische Polymere werden dagegen bevorzugt als fertige Matrix zur Inkubation mit den Stammzellen vorgelegt. Als Matrixmaterialien sind z. B. natürlich vorkommende Biopolymere, wie Collagen, Gelatine, Alginat, Agarose, Laminin, Fibronectin, Fibrin, Matrigel, Polysaccharide, Glycosaminoglyklanen oder Proteoglykane, geeignet, aber auch synthetische Polymere, wie z. B. Poly-? -Hydroxysäuren, Polycarbonat, Polyamide, Polyanhydride, Polyester, Polyurethane, Polyglycole, Polyoxyethylen, Polyoxypropylen, Polyacrylate, Polymethacrylate, Polycyanoacrylate, Polyacetale, Polyvinylchloride, flouride, -imidazole, Polyhydroxyvinyle, Polyvinylacetate, Polyethylenvinylacetate, chlorosulfinierte Polyolefine, Polyanhydride, Polyorthoester, Polycaprolactone, Polylactide, Polyphosphine, Milchsäure-Polymerisate oder Glycolsäure-Polymerisate. Es können auch Copolymere dieser Verbindungen oder andere Copolymere, wie z. B. Polylactid-co-glycolid oder Polyethylenglykol-Polylaktid , oder Mischungen dieser Polymere, bevorzugt Fibrin-Collagen, Fibrin-Alginat-, Alginat-Collagen-Mischungen. Die Polymere Matrix kann dabei als 2-dimensionaler Film oder als 3-dimensionaler-Körper aus einem oder mehrerer dieser Polymere ausgebildet sein.

Die adhäsiv bzw. immobilisierend wirkende polymere Matrix kann auch weitere Trägermaterialien umfassen, so z. B. poröse Träger, wie Hydroxyapatite oder Zeolithe. Aber auch polymere Trägermaterialen sind verwendbar, die mit Adhäsionsmolekülen beladen werden können, so daß eine selektive Anlagerung der Zellen auf der polymeren Matrix mit den mesenchymalen Stammzellen oder der von ihnen abgeleiteten Zellen zustande kommt.

Unter den gegebenen Bedingungen wachsen die mesenchymalen Stammzellen auf der vorgegebenen adhäsiven Oberfläche. Feste Polymermatrizen ergeben somit vorgeformte Zellkonstrukte, elastische oder gelartige Matrizen ergeben dagegen flexible Zellkonstrukte.

Die Matrix und/oder das Medium können weitere Zusatzstoffe, insbesondere bioaktive Faktoren, wie z. B. Wachstumsfaktoren, Wachstumshemmer, Hormone, Arzneimittelwirkstoffe, insbesondere Antibiotika, Cytostatika oder entzündungshemmende Stoffe, selektiv wirkende Zelladhäsiva, die Proliferation oder Differenzierung induzierende und fördernde bioaktive Faktoren, Vaskularisierungsfaktoren oder Immunmodulatoren, enthalten. Bevorzugt sind bioaktive Faktoren die das Wachstum und die Differenzierung der mesenchymalen Stammzellen nicht oder nur wenig beeinträchtigen. So können z. B. neben wachstumsfördernden Faktoren auch spezifisch epitheliale Stammzellen hemmende Substanzen zugesetzt werden.

Bevorzugt sind die bioaktiven Faktoren kovalent oder adsorptiv an die polymere Matrix oder an zusätzlich eingebrachte Trägermaterialien, insbesondere an poröse Träger gebunden.

Es können zum einen Zellkonstrukte enthaltend mesenchymale Stammzellen aber auch Zellkonstrukte enthaltend differenzierte Bindegewebszellen hergestellt werden.

Die Differenzierung der mesenchymalen Stammzellen aus Haarfollikeln zu Bindegewebszellen für die Herstellung solcher Bindegewebszellkonstrukten kann im Wachstumsmedium vor Inkubation der polymeren Matrix oder nach Inkubation direkt im Zellkonstrukt erfolgen.

Bevorzugt werden Zellkonstrukte, die mesenchymalen Stammzellen stammend aus Haarfollikeln enthalten unter Verwendung geeigneter Differenzierungsfaktoren und Kultivierungsbedingungen im matrixfixierten Zustand in daraus ableitbare Bindegewebszellkonstrukte umgewandelt.

Die Differenzierung kann dabei in vitro oder nach Reimplantation des Zellkonstrukts in vivo erfolgen. Im letzteren Fall werden die Differenzierungsfaktoren bevorzugt auf der polymeren Matrix angebracht. Für die in vivo Differenzierung können allerdings auch endogen vorhandene Differenzierungsfaktoren ausreichend sein, so daß auch Zellkonstrukte aus mesenchymalen Stammzellen aus Haarfollikeln ohne weitere Zusatzstoffe implantiert werden können.

Die Differenzierung kann dabei gemäß dem Fachmann bekannter Verfahren erfolgen. So sind z. B. Verfahren zur Herstellung von differenzierten Bindegewebszellen, wie z. B. Knochen- oder Knorpelzellen aus US 5,486,359, US oder aus 5,827,740 bekannt.

Zur Herstellung von Zellkonstrukten aus Fettgewebe werden z. B. die selektionierten mesenchymalen Stammzellen aus Haarfollikel mit einem FGF enthaltenden Medium auf einer polymeren Matrix ausgesät und kultiviert. Danach werden die Zellen einige Tage, bevorzugt unter serumfreien Bedingungen, in einem geeigneten Medium, z. B. DMEM/Hams F12 mit FGF gegebenenfalls unter Zusatz weiterer Faktoren, gehalten, wobei bevorzugt anfänglich Retinsäure zugesetzt wird. Nach Erlangen der Konfluenz können die Zellen mit 3-Isobutyl-1-methylxanthin (IBMX), Hydrocortison, Indomethacin oder einer Mischung daraus differenziert werden.

Das Arbeiten unter serumfreien Differenzierungsbedingungen ist dabei von Vorteil, da im Blutserum neben Albumin noch einige weitere Faktoren enthalten sind, die eine problemlose und effektive Differenzierung zu Fettzellen hemmen können.

Zur Herstellung von Zellkonstrukten aus Knorpelzellen werden z. B. die selektionierten und an eine polymere Matrix immobilisierten mesenchymalen Stammzellen aus Haarfollikeln in Gegenwart von DMEM mit Insulin, Dexamethason und/oder T3 zu Chondrozyten differenziert.

Die erfindungsgemäßen Zellkonstrukte können vor allem zur patientenschonenden Herstellung von patientenspezifischen Transplantaten verwendet werden. Die Verwendung der Zellkonstrukte gemäß dem Hauptanspruch zur Herstellung von Transplantaten ist somit ein weiterer Gegenstand der vorliegenden Erfindung.
Dabei können je nach Bedarf formstabile Konstrukte unter Verwendung von festen polymeren Matrizen oder formflexible Konstrukte unter Verwendung von elastischen oder gelförmigen polymeren Matrizen erzeugt werden.

In einer speziellen Ausführungsform besteht die polymere Matrix aus biokompatiblen und bioabbaubaren Materialien, so daß nach einer Implantation des Zellkonstruktes keine unerwünschten Reaktionen des Körpers erzeugt werden und nur die gewünschten differenzierten Gewebezellen übrigbleiben.

Mit der Fixierung weiterer Zusatzstoffe, wie z. B. von Immunsuppressiva oder entzündungshemmenden Wirkstoffen, im Transplantat können die Erfolgsaussichten einer Transplantation weiter erhöht werden. Auch Faktoren die eine Geweberegeneration fördern, können zusätzlich im Transplantat enthalten sein, insbesondere um die Einbettung des Transplantates in das umliegende Gewebe zu fördern.

Die erfindungsgemäßen Zellkonstrukte können darüber hinaus als Ausgangsprodukte zur Herstellung gentechnisch veränderter Zellen verwendet werden. Von besonderem Interesse sind hier die Transfektion von Zellen mit Genen, die Wachstumsfaktoren, wie z. B. von IGF, TGF-beta, oder von Hormonen, wie z. B. Insulin oder des Parathyroid-Hormons, exprimieren oder regulieren. Von besonderem Interesse ist auch die Transfektion mit Genen, die Gendefekte kompensieren können und somit Krankheiten lindern können. So können die transformierten Zellkonstrukte pharmazeutische Wirkstoffe nach einer Implantation produzieren und freisetzen.

Ein weiterer Gegenstand der vorliegenden Erfindung ist die Verwendung der erfindungsgemäßen Zellkonstrukte zur Herstellung von analytischen oder diagnostischen Assays. Unter Verwendung von mesenchymalen Stammzellen enthaltenden Zellkonstrukten kann z. B. ein Assay zur Identifikation geeigneter Differenzierungsfaktoren und Bedingungen betrieben werden bzw. die Wirkung bereits Bekannter testen. Zellkonstrukte, die bereits stark differenzierte Zelltypen enthalten, werden dagegen bevorzugt in Assays zur Identifikation geeigneter Arzneimittelwirkstoffe eingesetzt.
Im folgenden werden zur Verdeutlichung der Erfindung einige Ausführungsbeispiele beschrieben.

### Beispiel 1: Kultivierung von Zellen des Haarfollikels

Die Kopfhaut gesunder Probanden wurde mit 70 % Ethanol desinfiziert und mit einem mit PBS getränktem Pflaster (10 min) aufgeweicht. Mit einer Pinzette wurden 10-20 Kopfhaare langsam gezupft und in DMEM + 10 % FCS + Penicillin/Streptomycin gesammelt. Die anagenen Haare mit deutlich sichtbarem Haarfollikel werden selektiert und das Haar über dem Haarfollikel abgeschnitten. Die Haarfollikel werden kurz in einer Penicillin/Streptomycin-Lösung gebadet, mit Dispase (50 U/ml) behandelt und anschließend in eine mit Matrigel beschichtete 12-well Platte überführt. Um die für das Auswachsen nötige Adhärenz der Follikel an das Matrigel zu gewährleisten, wurden die Follikel mit einem Deckgläschen beschwert. In Gegenwart von DMEM, 20% FCS, bFGF (10 ng/ml), Retinsäure (1µM), 3 x nicht essentielle Aminosäuren , IGF-I (50 ng/ml), Hydrocortison (10 ng/ml), Pyruvat (1 mM), T3 (0.1 nM), Transferrin (6.25 µg/ml), Selen (6.25 µg/ml) und Glucagon (1 µg/ml) (im folgenden als Kulturmedium bezeichnet) zugesetzt wurden die Haarfollikel bei 37°C in feuchter Atmosphäre mit 5% CO₂ kultiviert. Nach ca. 4 Wochen wuchsen die Follikel aus, so dass die Kulturen nach ca. 2 weiteren Wochen konfluent waren. Zur Subkultivierung wurden die Zellen mit 0.1 % Collagenase abgelöst und in obigem Medium propagiert.

### Beispiel 2: Isolierung mesenchymaler Stammzellen aus Haarfollikelzellen

Die Selektion auf MSC erfolgt über Antikörper gekoppelte magnetische bead Separation entweder als Positiv- oder als Negativ-Selektionstechnik. Bei der Positiv-Selektion werden spezifische Oberflächenmoleküle der mesenchymalen Stammzellen gesucht, die auch bei mesenchymalen Stammzellen aus Haarfollikel angewendet werden können. Antikörper gegen CD13, CD49e, CD29, CD105 und smooth-muscle-alpha-actin erwiesen sich als geeignet. Mit Hilfe dieser Antikörper wurde über eine immunomagnetische Separation mit Dynabeads eine hochreine Population mesenchymaler Stammzellen aus Haarfollikeln hergestellt.
Um Veränderungen der Zellen bedingt durch das Binden der Antikörper bei der Positiv-Selektion zu verhindern, wurde parallel auch eine Negativ-Selektion mit anti CD24- und anti Cytokeratin 16-Antikörper durchgeführt.

Die gewonnenen Zellpopulationen der mesenchymalen Stammzellen waren bei beiden Selektionsmöglichkeiten in der Morphologie homogen und entsprachen morphologisch einem Fibroblasten-ähnlichen Zelltyp.

### Beispiel 3: Herstellung von 3-dimensionalen Transplantaten in Collagenmatrices

In einer 24-well wurden 120 µl Collagen (16 mg/ml 0.05% Eisessig) mit 70 µl 3x konzentriertem MEM-Medium , 40 µl FCS und 19 µl 0.1 N NaOH und 1x 10⁵ der selektionierten mesenchymalen Stammzellen aus Haarfollikel (150 µl) vermischt. Bei 37 °C gelierte die Collagen-Matrix innerhalb von 10 min. Die Kultivierung der Zellen in der Collagenmatrix erfolgte mit Kulturmedium. Die Zellen nahmen in dieser Matrix eine fibroblastäre Morphologie an.

### Beispiel 4: Herstellung von 3-dimensionalen Transplantaten in Alginatmatrices

Low viscosity Alginat (1.0 % in 0.9 % NaCl) wurde mit 4x 10⁵/ml der selektionierten mesenchymalen Stammzellen aus Haarfollikel vermischt und auf ein coverslip pipettiert. Nach vorsichtigem Eintauchen in 100 mM CaCl₂ gelierte Alginat. Das Alginatgel wurde danach mehrmals mit 0.9 % NaCl gewaschen. Die Kultivierung der Zellen erfolgte in Kulturmedium. Da Alginat sowohl immobilisierend als auch adhäsiv auf die Zellen wirkt, ist Alginat besonders gut für die Herstellung von Knorpel-Zellkonstrukten geeignet.

### Beispiel 5: Herstellung von 3-dimensionalen Transplantaten in Fibrinmatrices

25 µl Fibrinogen (21 mg/ml) wurde mit 100 µl 300 mM CaCl₂ und selektionierten mesenchymalen Stammzellen aus Haarfollikel (5 x 10⁵/ml) vermischt. Die Gelbildung wurde mit 4 U Thrombin gestartet und war nach 10 min bei 37 °C abgeschlossen. Die Kultivierung der Zellen erfolgte in Kulturmedium. Fibrinmatrices eigneten sich besonders gut zur Herstellung von Zellkonstrukten mit Adipozyten.

### Beispiel 6: Differenzierung der mesenchymalen Stammzellen aus Haarfollikel zu Fettzellen.

Die selektionierten mesenchymalen Stammzellen aus Haarfollikel wurden in einer Dichte von 6000/cm² ausgesät und in Kulturmedium 2 Tage kultiviert. Danach wurden die Zellen 7 Tage unter serumfreien Bedingungen in DMEM/Ham s F12 3:1 + Panthotenat (4 µg/ml) + Transferrin (2 µg/ml) + Biotin (1µM) + Insulin (6 µg/ml) + bFGF (10 ng/ml), Heparin-Natrium 5000 (20 µg/ml) gehalten, wobei in den ersten 5 Tagen Retinsäure (1 µM) zugesetzt wurde. Nach Erlangen der Konfluenz wurden die Zellen mit 500 µM IBMX und 100 nM Hydrocortison, Indomethacin (100 µM) differenziert. Mit einer Färbung der Fetttropfen mit Oil Red O konnte gezeigt werden, dass ca. 40 % der Zellen zu Adipocyten differenziert sind.

### Beispiel 7: Differenzierung der selektionierten mesenchymalen Stammzellen aus Haarfollikeln zu Knorpelzellen.

Die selektionierten mesenchymalen Stammzellen aus Haarfollikeln wurden in Gegenwart von DMEM mit Insulin, Dexamethason und T3 zu Chondrozyten differenziert. Der Differenzierungserfolg wurde mit einer Safranin-O-Färbung überprüft.

## Patentansprüche

1. Zellkonstrukte enthaltend eine Polymermatrix und mesenchymale Stammzellen aus Haarfollikeln oder daraus ableitbarer Zelltypen des Bindegewebes.

2. Zellkonstrukte gemäß Anspruch 1 **dadurch gekennzeichnet, daß** die ableitbaren Zelltypen des Bindegewebes Knochen-, Knorpel-, Muskel-, Fettgewebe-, Bänder- Sehnen- oder Dermiszellen sind.

3. Zellkonstrukte gemäß einem der vorhergehenden Ansprüche, **dadurch gekennzeichnet, daß** die Polymermatrix biokompatibel und bioabbaubar ist.

4. Zellkonstrukte gemäß einem der vorhergehenden Ansprüche, **dadurch gekennzeichnet, daß** die Polymermatrix Collagen, Alginat, Agarose, Gelatine, Laminin, Fibronectin, Fibrin, Matrigel, Polysaccharide, Glycosaminoglykane, Proteoglykane, Poly-α-Hydroxysäuren, Polycarbonat, Polyamide, Polyanhydride, Polyestern, Polyurethane, Polyglycole, Polylactid-co-glycolid oder Polyethylenglykol-Polylaktid, Polyoxyethylen, Polyoxypropylen, Polyacrylate, Polymethacrylate, Polycyanoacrylate, Polyacetale, Polyvinylchloride, -flouride, -imidazole, Polyhydroxyvinyle, Polyvinylacetat, Polyethylenvinylacetate, chlorosulfinierte Polyolefine, Polyanhydride, Polyorthoester, Polycaprolactamen, Polylactide, Polyphosphine, Milchsäure-Polymerisate, Glycolsäure-Polymerisate, Copolymere dieser Verbindungen oder Mischungen dieser Polymereenthält.

5. Zellkonstrukte gemäß einem der vorherigen Ansprüche, **dadurch gekennzeichnet, daß** zusätzliche Trägermaterialien enthalten sind.

6. Zellkonstrukte gemäß einem der vorhergehenden Ansprüche enthaltend weitere Zusatzstoffe.

7. Zellkonstrukte gemäß dem vorherigen Anspruch, **dadurch gekennzeichnet, daß** als Zusatzstoffe Wachstumsfaktoren, Wachstumshemmer. Hormone, Arzneimittelwirkstoffe, selektiv wirkende Zelladhäsiva, die Proliferation oder Differenzierung induzierende und fördernde bioaktive Faktoren, Vaskularisierungsfaktoren und/oder Immunmodulatoren enthalten sind.

8. Verfahren zur Reinigung von mesenchymalen Stammzellen aus gezupften Haarfollikeln umfassend folgende Verfahrensschritte:
a) Separieren der gezupften Haarfollikel vom Resthaar, ,
b) Überführen der Haarfollikel auf eine feste oder gelförmige Oberfläche,
c) Inkubation mit einem FGF enthaltenden Medium bei einer Temperatur zwischen 25 und 40°C,
d) Herstellen einer Einzelzellsuspension durch Ablösen der ausgewachsenen Haarfollikel von der Oberfläche
e) Propagierung der mesenchymalen Stammzellen in einem geeigneten Medium.

9. Verfahren gemäß Anspruch 8, **dadurch gekennzeichnet, daß** Changs-Medium oder DMEM, DMEM/Ham's F12, M199, E199, MEM oder EMEM als Medium zur Kultivierung der mesenchymalen Stammzellen verwendet wird.

10. Verfahren gemäß einem der Ansprüche 8 oder 9, **dadurch gekennzeichnet, daß** dem Medium zusätzlich bFGF, Serum, IGF-I, Insulin, Hydrocortison, Pyruvat. Retinsäure, nicht essentielle Aminosäuren, Triiodthyronin (T3), Transferrin, Selen und/oder Glucagon zugesetzt wird.

11. Verfahren gemäß einem der Ansprüche 8 bis 10, **dadurch gekennzeichnet, daß** dem Medium Differenzierungsfaktoren zugesetzt werden.

12. Verfahren nach einem der Ansprüche 8 bis 11, **dadurch gekennzeichnet, daß** eine Selektion der mesenchymalen Stammzellen mittels FACS, mittels immunomagnetischer Verfahren oder mittels Percoll-Dichtegradientenzentrifugation erfolgt.

13. Verfahren nach dem vorherigen Anspruch, **dadurch gekennzeichnet, daß** eine weitere Reinigung mittels immunologischer Negativ-Selektion unter Verwendung von Antikörpern gegen die epithelspezifischen Antigene CD24 oder Cytokeratin 16 erfolgt.

14. Homogene Zellkultur aus mesenchymalen Stammzellen aus Haarfollikeln erhältlich nach einem Verfahren gemäß der Ansprüche 8 bis 10 und zusätzlicher Aufreinigung mittels FACS, mittels immunomagnetischer Verfahren oder mittels immunologischer Negativ-Selektion.

15. Verfahren zur Herstellung von Zellkonstrukten gemäß Anspruch 1, **dadurch gekennzeichnet, daß** eine auf mesenchymale Stammzellen immobilisierend bzw. adhäsiv wirkende polymere Matrix mit einer Suspension enthaltend mesenchymale Stammzellen aus Haarfollikeln oder daraus differenzierten Bindegewebszellen inkubiert wird.

16. Verfahren gemäß dem vorherigen Anspruch, **dadurch gekennzeichnet, daß** die Polymerisation vor der Inkubation und/oder das Gelieren der Matrix vor oder während der Inkubation erfolgt.

17. Verfahren gemäß dem vorherigen Anspruch, **dadurch gekennzeichnet, daß** die Zellsuspension und/oder die polymere Matrix Differenzierungsfaktoren enthält.

18. Verfahren gemäß dem vorherigen Anspruch, **dadurch gekennzeichnet, daß** durch Verwendung von Differenzierungsfaktoren die mesenchymalen Stammzellen des Haarfollikels in mehr als einen Bindegewebetyp in vitro differenzieren.

19. Verwendung von Zellkonstrukten gemäß einem der Ansprüche 1 bis 7 zur Herstellung von Transplantaten zur Behandlung von Knochen-, Knorpel-, Muskel-, Fettgewebe-, Dermis-, Bänder- oder Sehnenschäden, -verlust oder -krankheiten.

20. Verwendung von mesenchymalen Stammzellen aus Haarfollikeln, daraus differenzierten Bindegewebszellen oder von Zellkonstrukten gemäß einem der Ansprüche 1 bis 7 zur Herstellung oder Durchführung diagnostischer oder analytischer Assays.

21. Verwendung von mesenchymalen Stammzellen aus Haarfollikeln, daraus differenzierten Bindegewebszellen oder von Zellkonstrukten gemäß dem vorherigen Anspruch zum Screening von Substanzen, die auf mesenchymale Stammzellen, Knochenzellen, Knorpelzellen, Muskelzellen, Fettgewebezellen, Dermiszellen oder sonstigen Bindegewebszellen biologisch aktiv wirken.

22. Verwendung von mesenchymalen Stammzellen aus Haarfollikeln, daraus differenzierten Bindegewebszellen oder von Zellkonstrukten gemäß den beiden vorherigen Ansprüchen zur Identifizierung von geeigneten Arzneimittelwirkstoffen.

23. Verwendung von mesenchymalen Stammzellen aus Haarfollikeln oder teilungsfähigen Vorläuferzellen von differenzierten Bindegewebszellen oder von Zellkonstrukten gemäß einem der Ansprüche 1 bis 7 zur Herstellung gentechnisch veränderter Zellen oder Zellkonstrukte.

## Claims

1. Cell constructs comprising a polymer matrix and hair follicle-derived mesenchymal stem cells or connective tissue cell types which can derived therefrom.

2. Cell constructs according to Claim 1, **characterized in that** the derivable connective tissue cell types are bone, cartilage, muscle, fat tissue, ligament, tendon or skin cells.

3. Cell constructs according to one of the preceding claims, **characterized in that** the polymer matrix is biocompatible and biodegradable.

4. Cell constructs according to one of the preceding claims, **characterized in that** the polymer matrix comprises collagen, alginate, agarose, gelatin, laminin, fibronectin, fibrin, matrigel, polysaccharides, glycosaminoglycans, proteoglycans, poly-α-hydroxy acids, polycarbonate, polyamides, polyanhydrides, polyesters, polyurethanes, polyglycols, polylactide-co-glycolide or polyethylene glycol polylactide, polyoxyethylene, polyoxypropylene, polyacrylates, polymethacrylates, polycyanoacrylates, polyacetals, poly(vinyl chlorides), poly(vinyl fluorides), poly(vinylimidazoles), poly(hydroxyvinyls), poly(vinyl acetate), poly(ethylene vinyl acetates), chlorosulphinated polyolefins, polyanhydrides, polyorthoesters, polycaprolactams, polylactides, polyphosphines, lactic acid polymers, glycolic acid polymers, or copolymers of these compounds, or mixtures of these polymers.

5. Cell constructs according to one of the preceding claims, **characterized in that** additional carrier materials are present.

6. Cell constructs according to one of the preceding claims comprising further additives.

7. Cell constructs according to the preceding claim, **characterized in that** the additives present are growth factors, growth inhibitors, hormones, pharmaceutical active compounds, cell adhesives having a selective action, bioactive factors which induce and promote proliferation or differentiation, vascularization factors and/or immunomodulators.

8. Method for purifying mesenchymal stem cells from plucked hair follicles, comprising the following procedural steps:
a) separating the plucked hair follicles from the residual hair,
b) transferring the hair follicles to a solid or gelatinous surface,
c) incubating with an FGF-containing medium at a temperature between 25 and 40°C,
d) preparing a single cell suspension by detaching the grown hair follicles from the surface
e) propagating the mesenchymal stem cells in a suitable medium.

9. Method according to Claim 8, **characterized in that** Chang's medium or DMEM, DMEM/Ham's F12, M199, E199, MEM or EMEM is used as the medium for culturing the mesenchymal stem cells.

10. Method according to Claim 8 or 9, **characterized in that** bFGF, serum, IGF-1, insulin, hydrocortisone, pyruvate, retinoic acid, non-essential amino acids, triiodothyronine (T3), transferrin, selenium and/or glucagon is/are added to the medium.

11. Method according to one of Claims 8 to 10, **characterized in that** differentiation factors are added to the medium.

12. Method according to one of Claims 8 to 11, **characterized in that** the mesenchymal stem cells are selected by means of FACS, by means of immunomagnetic methods or by means of Percoll density gradient centrifugation.

13. Method according to the preceding claim, **characterized in that** a further purification is effected by means of immunological negative selection using antibodies directed against the epithelium-specific antigens CD24 or cytokeratin 16.

14. Homogeneous cell culture which is composed of hair follicle-derive mesenchymal stem cells and which can be obtained by a method according to Claims 8 to 10 and additional purification by means of FACS, by means of immunomagnetic methods or by means of immunological negative selection.

15. Method for preparing cell constructs according to Claim 1, **characterized in that** a polymeric matrix which has an immobilizing or adhesive effect on mesenchymal stem cells is incubated with a suspension containing hair follicle-derived mesenchymal stem cells or connective tissue cells which are differentiated therefrom.

16. Method according to the preceding claim, **characterized in that** the polymerization is effected prior to the incubation and/or the gelation of the matrix is effected prior to or during the incubation.

17. Method according to the preceding claim, **characterized in that** the cell suspension and/or the polymeric matrix contain(s) differentiation factors.

18. Method according to the preceding claim, **characterized in that**, as a result of using differentiation factors, the mesenchymal stem cells of the hair follicle differentiate in vitro into more than one connective tissue type.

19. Use of cell constructs according to one of Claims 1 to 7 for preparing transplants for treating bone, cartilage, muscle, fat tissue, skin, ligament or tendon damage, loss or diseases.

20. Use of hair follicle-derived mesenchymal stem cells, connective tissue cells which are differentiated therefrom, or of cell constructs according to one of Claims 1 to 7 for preparing or implementing diagnostic or analytical assays.

21. Use of hair follicle-derived mesenchymal stem cells, connective tissue cells which are differentiated therefrom or of cell constructs according to the preceding claim for screening substances which act in a biologically active manner on mesenchymal stem cells, bone cells, cartilage cells, muscle cells, fat tissue cells, skin cells or other connective tissue cells.

22. Use of hair follicle-derived mesenchymal stem cells, connective tissue cells which are differentiated therefrom or of cell constructs according to the two preceding claims for identifying suitable pharmaceutical active compounds.

23. Use of hair follicle-derived mesenchymal stem cells or division-capable precursor cells of differentiated connective tissue cells or of cell constructs according to one of Claims 1 to 7 for preparing recombinantly altered cells or cell constructs.

## Revendications

1. Constructions cellulaires contenant une matrice polymère et des cellules souches mésenchymateuses de follicules capillaires ou des types cellulaires du tissu conjonctif dérivables de celles-ci.

2. Constructions cellulaires selon la revendication 1, **caractérisées en ce que** les types cellulaires du tissu conjonctif dérivables sont des cellules d'os, de cartilage, de muscle, de tissu gras, de ligaments, de tendons ou de derme.

3. Constructions cellulaires selon l'une des revendications précédentes, **caractérisées en ce que** la matrice polymère est biocompatible et biodégradable.

4. Constructions cellulaires selon l'une des revendications précédentes, **caractérisées en ce que** la matrice polymère contient du collagène, de l'alginate, de l'agarose, de la gélatine, de la laminine, de la fibronectine, de la fibrine, du matrigel, des polysaccharides, des glycosaminoglycanes, des protéoglycanes, des poly-α-hydroxyacides, du polycarbonate, des polyamides, des polyanhydrides, des polyesters, des polyuréthanes, des polyglycols, du polylactide-co-glycolide ou du polyéthylèneglycol-polylactide, du polyoxyéthylène, du polyoxypropylène, des polyacrylates, des polyméthacrylates, des polycyanoacrylates, des polyacétales, des polychlorures de vinyle, des polyfluorures de vinyle, des polyimidazoles de vinyle, des polyhydroxyvinyles, du polyacétate de vinyle, du polyéthylènacétate de vinyle, des polyoléfines chlorosulfonées, des polyanhydrides, des polyorthoesters, des polycaprolactames, des polylactides, des polyphosphines, des polymérisats d'acide lactique, des polymérisats d'acide glycolique, des copolymères de ces composés ou des mélanges de ces polymères.

5. Constructions cellulaires selon l'une des revendications précédentes, **caractérisées en ce qu'**elles contiennent des matériaux véhicules supplémentaires.

6. Constructions cellulaires selon l'une des revendications précédentes, contenant d'autres additifs.

7. Constructions cellulaires selon la revendication précédente, **caractérisées en ce qu'**elles contiennent, en tant qu'additifs, des facteurs de croissance, des inhibiteurs de croissance, des hormones, des principes actifs médicaments, des adhésifs cellulaires agissant sélectivement, des facteurs bioactifs induisant et encourageant la prolifération ou la différenciation, des facteurs de vascularisation et/ou des immunomodulateurs.

8. Procédé pour la purification de cellules souches mésenchymateuses à partir de follicules capillaires détachés comprenant les étapes suivantes de procédé :
a) Séparation des follicules capillaires du reste de la chevelure,
b) Transfert des follicules capillaires sur une surface solide ou sous forme de gel,
c) Incubation avec un milieu contenant du FGF à une température entre 25 et 40°C,
d) Préparation d'une suspension de cellules individuelles par décollement de la surface des follicules capillaires ayant poussé,
e) Propagation des cellules souches mésenchymateuses dans un milieu approprié.

9. Procédé selon la revendication 8, **caractérisé en ce que** l'on utilise, en tant que milieu pour la culture des cellules souches mésenchymateuses, le milieu de Chang, le DMEM, DMEM/Ham, F12, M199, E199, MEM ou EMEM.

10. Procédé selon l'une des revendications 8 à 9, **caractérisé en ce que** l'on ajoute en plus au milieu du FGF, du sérum, de l'IGF-1, de l'insuline, de l'hydrocortisone, du pyruvate, de l'acide rétinoique, des acides aminés non essentiels, de la triiodothyronine (T3), de la transferrine, du sélénium et/ou du glucagon.

11. Procédé selon l'une des revendications 8 à 10, **caractérisé en ce que** l'on ajoute au milieu des facteurs de différenciation.

12. Procédé selon l'une des revendications 8 à 11, **caractérisé en ce que** l'on réalise une sélection des cellules souches mésenchymateuses au moyen d'un FACS, au moyen de procédés immunomagnétiques ou au moyen d'une centrifugation de gradients de densité de Percoll.

13. Procédé selon la revendication précédente, **caractérisé en ce que** l'on réalise une autre purification au moyen d'une sélection négative par l'utilisation d'anticorps dirigés contre les antigènes spécifiques de l'épithélium CD24 ou cytokératine 16.

14. Culture cellulaire homogène de cellules souches mésenchymateuses de follicules de cheveux susceptible d'être obtenue par un procédé selon les revendications 8 à 10 et une purification supplémentaire au moyen d'un FACS, au moyen de procédés immunomagnétiques ou au moyen d'une sélection négative immunologique.

15. Procédé pour la préparation de constructions cellulaires selon la revendication 1, **caractérisé en ce qu'**une matrice polymère, sur laquelle peuvent être immobilisées ou peuvent adhérer des cellules souches mésenchymateuses, est incubée avec une suspension contenant des cellules souches mésenchymateuses de follicules capillaires ou des cellules du tissu conjonctif différenciées à partir de celles-ci.

16. Procédé selon la revendication précédente **caractérisé en ce que** la polymérisation avant l'incubation et/ou la gélification de la matrice a lieu avant ou pendant l'incubation.

17. Procédé selon la revendication précédente, **caractérisé en ce que** la suspension cellulaire et/ou la matrice polymère contient des facteurs de différenciation.

18. Procédé selon la revendication précédente, **caractérisé en ce que**, par l'utilisation de facteurs de différenciation, les cellules souches mésenchymateuses du follicule capillaire se différencient in vitro en plus d'un type du tissu conjonctif.

19. Utilisation de constructions cellulaires selon l'une des revendications 1 à 7 pour la préparation de transplants pour le traitement de dommages, de la perte ou de maladies de l'os, du cartilage, du muscle, du tissu gras, du derme, des ligaments ou des tendons.

20. Utilisation de cellules souches mésenchymateuses de follicules capillaires, de cellules du tissu conjonctif différenciées à partir de celles-ci ou de constructions cellulaires selon l'une des revendications 1 à 7 pour la préparation ou la mise en oeuvre de tests diagnostics ou analytiques.

21. Utilisation de cellules souches mésenchymateuses de follicules capillaires, de cellules du tissu conjonctif différenciées à partir de celles-ci ou de constructions cellulaires selon la revendication précédente pour le criblage de substances qui sont biologiquement actives sur les cellules souches mésenchymateuses, les cellules osseuses, les cellules cartilagineuses, les cellules musculaires, les cellules du tissu gras, les cellules dermiques ou d'autres cellules du tissu conjonctif.

22. Utilisation de cellules souches mésenchymateuses de follicules capillaires, de cellules du tissu conjonctif différenciées à partir de celles-ci ou de constructions cellulaires selon les deux revendications précédentes pour l'identification de principes actifs de médicaments appropriés.

23. Utilisation de cellules souches mésenchymateuses de follicules capillaires ou de cellules précurseurs de cellules du tissu conjonctif différenciées capables de se diviser ou de constructions cellulaires selon l'une des revendications 1 à 7 pour la préparation de cellules ou constructions cellulaires modifiées par génie génétique.
